# EUROPEAN PATENT SPECIFICATION

(11) **EP 1 194 099 B1**
(45) Date of publication and mention of the grant of the patent: **01.10.2003**
(21) Application number: 00947903.1
(22) Date of filing: 30.06.2000
(51) Int. Cl.: A61F 13/20, A61F 13/22

(54) **TAMPON FOR FEMININE HYGIENE AND PROCESS AND APPARATUS FOR ITS PRODUCTION**
TAMPON MIT EINEM PERFORIERTEN FILMÜBERZUG WÄRMEVERBUNDEN AN EINE ABSORBIERENDE FASERSCHICHT
TAMPON HYGIENIQUE, PROCEDE ET APPAREIL DE PRODUCTION ASSOCIES

(30) Priority: 30.06.1999 US 141690 P; 29.06.2000 US 606559
(43) Date of publication of application: 10.04.2002
(73) Proprietor: JOHNSON & JOHNSON GmbH, 40474 Düsseldorf (DE)
(72) Inventor: SCHOELLING, Hans-Werner, D-58252 Ennepetal (DE)
(74) Representative: Groening, Hans Wilhelm, Dipl.-Ing.
(86) International application number: EP0006136
(87) International publication number: WO01001908

(56) References cited:
- WO-A-94/12328
- WO-A-97/23398
- WO-A-98/53966
- WO-A-99/00096
- DE-A- 3 519 514
- DE-A- 3 519 515
- DE-U- 8 802 003
- US-A- 3 183 910
- US-A- 4 095 542
- US-A- 4 129 679
- US-A- 4 816 100
- US-A- 5 363 728
- US-A- 5 750 446

## Description

### Field of the Invention

The invention relates to process for forming a tampon useful in feminine hygiene, the tampon itself, and an apparatus for its production.

### Background of the Invention

A tampon disclosed in Friese, US Pat. No. 4,816,100 (DE 33 47 649 C2) comprises a section of specific length of a fleece web composed of a mixture of randomly oriented natural and artificial fibers and having a width corresponding approximately to the length of the tampon, the fleece web essentially being wound up on itself to form a tampon blank which is provided with a withdrawal cord and which is pressed essentially radially into the final shape of the tampon.

In the case of the tampon described above, and also in the case of other tampons according to the prior art, the end region of the fleece web, from which the tampon is wound, has proven to be a problem point. In order to ensure the most uniform possible, cylindrical surface of the tampon, in particular in order to avoid a step in the thickness of the fleece web at the point at which one end of the fleece web ends on the outer surface of the tampon, it is known, for example from DE 33 47 649 C2, to weaken the fleece web at the separation points at which it has to be divided in order to form the tampon sections, this being done by its cross section being reduced. The fleece web is subsequently severed at the weak point formed in this way, as a result of which the randomly oriented natural and artificial fibers are torn apart in the region of the weak point and are also partially pulled out of the fibrous composite, so that the cross-section of the fleece web and, respectively, the fleece web decreases as uniformly as possible and runs smoothly.

In consequence, as a rule a large number of individual fibers are located individually, and at least partially detached from the nonwoven fibrous composite, in the edge regions of the tampon sections. Following the operations of winding and pressing, the edge fibers, which are essentially aligned longitudinally, have the tendency to counteract the shaping exerted by the winding and pressing. At the same time, the fibers have a characteristic similar to a "memory effect", because of their stiffness.

This "memory effect" characteristic leads to those fibers which are in particular located in the edge region and are torn or exposed, being at least partially aligned tangentially to the circumference of the tampon, which has a negative influence on the external structure of the tampon and at least leads to an increased loading and possibly even to damage to the liquid-permeable wrapping material wound around the tampon.

This effect is to be observed in particular in the case of very stiff fibers, which exhibit an increased "memory tendency" and as a result partly project outward through the sheathing forming the wrapping material. There is the risk that such fibers lead to irritation of the user and/or leave the tampon assembly.

The document WO 97/23398 discloses a method and a device for perforating, cutting and/or scoring a continuous web of material such as paper cardboard or the like. According to the disclosed method and the device active means and counter pressure means are selectively moved into and out of a selectively active position, in which they exert pressure against each other and against the portion of the web lying therebetween during the movement of the web. The document discloses further rollers as such active means and counter pressure means, whereas these rollers comprise a blade member and a rod respectively, interacting with each other, thereby cutting or perforating the web.

Document WO 98/53966 discloses an apparatus and a method for selectively making a longitudinal perforation on web materials, whereas an apparatus according to the teaching of this documents comprises first and second perforator wheels and corresponding first and second anvil cylinders, wherein the cylinders are rotated synchronously with the web movement moving between the perforator wheels and the anvil cylinders.

US 4,816,100 discloses a feminine hygiene tampon and a method and an apparatus for making such a tampon, whereas the process for producing such a tampon comprises continuously feeding a nonwoven ribbon and weakening it transversely to its longitudinal direction for producing a length section necessary for the manufacture of the tampon and severing it at the weak point with formation of a nonwoven ribbon section to be rolled upon to give the tampon blank, being compressed to form the final tampon.

Document DE 35 19 515 A1 discloses a method for manufacturing a tampon, wherein a fleece web is provided and pre-cut over its width to form a fleece web segment, whereas later the segments are completely separated to form an individual fleece web to be wound up into a tampon blank, which is compressed to form the final tampon.

### Summary of the Invention

The present invention is therefore based on the object of producing a tampon having an improved surface structure. This improved structure permits the gentle and smooth insertion of the tampon into the body cavity and reduces the above-mentioned negative effects of the memory effect characteristic, in particular, of stiff fibers. At the same time, the absorbency, the absorption capacity and the expansion capacity of the tampon are not to be impaired, and in spite of these improvements, it should be possible to mass-produce the tampon maintaining a high quality of the tampon. It is therefore a further object of the invention to provide a process and an apparatus for producing such a tampon.

The tampon includes a section of a specific length of a nonwoven fibrous web section, a cover strip section thermally bonded to the one end of the nonwoven fibrous web section, and a withdrawal string extending from the tampon. The ends of the nonwoven fibrous web section are defined by a reduced cross-section, and the terminal edges of the ends being defined by alternating sections of cut fibers and extended fibers wherein the sections of the cut fibers amounts to at least about 70% of the width of the nonwoven fibrous web section. The cover strip section includes a liquid-permeable, at least partially thermoplastic material, and it extends beyond the one end of the nonwoven fibrous web section. The nonwoven fibrous web section is wound essentially upon itself to give a substantially round cylindrical tampon blank preform with the cover strip section extending beyond the one end of the nonwoven fibrous web section being thermally bonded to an overlapped portion of the cover strip section to maintain the tampon preform as a cylinder and wherein the cylindrical preform is radially pressed to form a substantially cylindrical compressed tampon.

An inventive process comprising the following steps can form the tampon. A continuous fibrous web is cut essentially over its width to form a fleece web segment connected to the continuous fibrous web through a plurality of uncut extensions; the fleece web segment is pulled away from the continuous fibrous web to tear the uncut extensions thereby forming an individual fleece web; the individual fleece web is formed into a tampon blank; and the tampon blank is compressed to form the tampon.

### Brief Description of the Drawing

FIG. 1 shows an embodiment of a cutting station according to the invention and a separated section of the fleece web for a tampon according to the invention, in a perspective view.
FIG. 2 shows a cross-section through a pair of cutting rolls, normal to their axis of rotation, of the embodiment shown in FIG. 1, in the region of knife cutters.
FIG. 3 shows a cross-section, analogous to FIG. 2, through the cutting rolls of the embodiment shown in FIG. 1 in the region of the discontinuous knife cutters.
FIG. 4 shows a wound-up section of an embodiment of a tampon according to the invention.
FIG. 5 shows an embodiment of a tampon according to the invention.

### Detailed Description of the Preferred Embodiment

According to the invention, a continuous fibrous web is cut essentially over its width to form a fleece web segment connected to the continuous fibrous web through a plurality of uncut extensions. The fleece web is then pulled away from the continuous fibrous web to tear the uncut extensions and to separate the fleece web segment. This provides a rather gradual diminution of the amount of fibers at the end of the fleece web segment, and it makes possible an optimum adaptation of the longitudinal ends of the fleece web, and hence of the surface of the wound and pressed tampon. This may be considered both with regard to a uniform structure of the surface of the tampon and also with regard to the suppressing the action of a "memory effect of the fibers located in the edge region of the fleece web. The cut fibers are arranged without significantly orienting them during the cutting process, and any orientation of fibers in the uncut extensions is minimized. Thus, any "memory effect" tending to cause individual, relatively rigid fibers to extend out of the fleece web does not occur. The fibers are held under control in the overall tampon blank and do not project from the outer surface of the essentially cylindrical tampon.

In addition, the number of exposed fibers and fibers partly pulled out of the fibrous composite is lower, because of the reduced tearing length over the overall width of the nonwoven section, so that any fibers that are pulled out of the fleece web segment that may, due to the "memory effect" load up on the wrapping material, are largely avoided. In addition, the force necessary to separate the individual fleece web segments from the continuous fibrous web is considerably reduced as a result of the cutting action. The force exerted on the individual fibers in the direction out of the fibrous composite by the tearing operation is reduced, so that the fibers are merely pulled out of the fibrous composite over a shorter distance.

The combination of cutting a substantial portion along the width of the continuous fibrous web and the tearing of the uncut extensions provides a thinning or weakening of the material in the end region of the section. Since individual fibers can also extend or be oriented in the direction of the cut or torn edges from the uncut extensions, it is possible that some of these fibers are distributed beyond the cut edge.

It is advantageous to alternate the cut portions and torn extensions, and preferably, the cut portions are arranged to account for at about least 70% of the width of the fibrous web. Preferably, the proportion of cut portions amounts to about 80% or 90% of the width. It is also preferred that the torn portions are distributed uniformly across the longitudinal ends of the fleece web. This achieves a gentle transition and a smooth and uniform surface of the tampon.

In a particularly preferred tampon, the section is additionally weakened at least at its outer longitudinal end which is implemented in particular by reducing the cross-section over portion of the fleece web near the cut end. This supports and further improves the uniform transition and the smooth surface of the tampon by providing a taper in the final winding of the tampon blank.

In one embodiment of the tampon according to the invention, the longitudinal ends of the fleece web essentially form a right angle with a longitudinal axis of the fleece web, and hence, the continuous web. The fleece web therefore essentially has a rectangular shape. However, it is also possible for the longitudinal ends of the section to form an oblique angle with the longitudinal axis of the section. This oblique angle is preferably about 85° to about 89°. The section to be produced can therefore be adapted by choice to a specific tampon design.

Furthermore, such a parallelogram-shaped fleece web has the advantage during production that during the cutting operation the pressure on the cutting devices can be lower, since the fleece web is not severed abruptly over its entire width, but it is cut in a shearing fashion, beginning at one longitudinal edge of the continuous fibrous web. A reduction of the pressure on the cutting devices generally also leads to lower wear and a longer service life. Finally, an oblique angle has the advantage that the outer terminating end of the fleece web is distributed over the cylindrical circumference of the tampon, which overall leads to a more uniform surface and therefore to the simplified insertion of the tampon into the body cavity.

In a preferred embodiment, the fibers of the fleece web have an irregular cross section, for example a multi-limbed cross-section. These fibers lead to an improved structure of the fleece web and to improved absorption or take-up properties, and therefore to an improved expansion characteristic of the tampon. Examples of these fibers are described in EP 301 874 and are sold as DANUFIL VY fibers, available from Acordis, Ltd.

The irregular cross sections of the fibers of the fleece web also leads to increased stiffness of the fibers. So that, in particular in the case of such preferred fibers being used, the tampon according to the invention is more helpful.

The tampon preferably has, at one and of the fleece web, a wrapping material which is fastened by heat sealing, is made of liquid-permeable, at least partly thermoplastic material, which is wound around the absorbent material and is joined to the outside of the fleece web by heat sealing. This wrapping material surrounds the circumferential surface of the tampon, so that a tampon cover is formed. The wrapping material and the fleece web are preferably joined to each other by thermally bonded joints arranged at intervals from one another. It is possible for these joints to have the shape of points and/or lines as described in commonly assigned, copending US Ser. No. , entitled "Tampon Having Apertured Film Cover Thermobonded to Fibrous Absorbent Structure" (Attorney Docket J&J-1925), the disclosure of which is herein incorporated by reference.

The wrapping material may comprise bicomponent fibers, fibers formed of at least two polymeric materials having a difference in melting points of 30°C or more. Preferred fibers have a polyester or polypropylene core and a sheath of polyethylene, preferably high density polyethylene ("HDPE"), which has a melting point lower than that of polyester and polypropylene.

The wrapping material may also comprise an apertured plastic film. Again, it is preferred that this material has at least two components with a melting point that differs by at least about 30°C. Examples of such films are disclosed in US Ser. Nos. 09/345,088 and 09/345,089, the disclosures of which are herein incorporated by reference.

In the case of a thin plastic film, and in particular in the case of the use of the above-mentioned advantageous, stiff, fibers which are irregular in cross section. A tampon according to the invention is very helpful, since end fibers are able to penetrate the holes of the plastic film because of the "memory effect", or they can be lifted off of the nonwoven material. This is reliably avoided by the structure of the tampon according to the invention.

A process similar to that of the present invention is disclosed in Friese, US Pat. No. 4,816,100. the disclosure of which is herein incorporated by reference. However, the present invention incorporates cutting the fleece web over essentially the entire width of the fleece web leaving a number of uncut extensions connecting the section to the continuous fleece web, and tearing apart the uncut extensions of successive fleece webs.

The process further also provides for weakening of the cross section at least of the rear end of a fleece web, which forms the outer end of the fleece web segment. This weakening of the fleece web end is carried out over a longitudinal section, for example by exerting pressure on the fleece web in this region.

The cutting and the weakening may be carried out approximately at a right angle to the longitudinal axis of the fleece web. However, the cutting and/or the weakening are preferably carried out at an oblique angle to the longitudinal axis of the fleece web, preferably about 85° to about 89°. In particular when knife rolls are used, but also in the case of all other cutting devices. The pressure on the cutting and weakening devices can be reduced by means of such a cutting and/or weakening direction, since the cutting and/or the weakening is carried out gradually and not simultaneously over the entire width of the fleece web. As a result, the process is significantly simplified, and in addition the loading of the tools needed for the process is reduced and their service life is increased.

The apparatus according to the invention comprises a cutting station with a pair of cutting rolls, a knife roll with at least one knife, and a backing roll, the at least one knife being arranged on at least one portion of an outer surface of the knife roll and having, at least in part, discontinuities for the webs of the fleece webs.

Such a cutting station can be used in the apparatus described in Friese, US Pat. No. 4,816,100. In one preferred embodiment, the knife on the outer surface of the knife roll is arranged essentially along a generatrix of the knife roll. As a result, an essentially rectangular fleece web is produced for the tampon, as described above.

In another preferred embodiment, the knife is arranged on the outer surface of the knife roll but at an angle with respect to a generatrix (i.e., at an oblique angle to the longitudinal axis of the fleece web), to produce a fleece web segment in the form of a parallelogram, as described above. The angle formed by the knife with the generatrix is preferably between about 1° and about 5°.

In an advantageous refinement, the cutting station also comprises a weakening device. This weakening device is formed as a press jaw, integral with the cutting station, by a thickening of the knife base extending over the entire width, and also comprises a region of the backing roll. During the cutting operation, a pressure is exerted on the longitudinal ends of the fleece web, which leads to a reduction in the cross section of the longitudinal ends. However, it is also possible to arrange the weakening device separately, upstream or downstream of the cutting rolls or the cutting station. The cutter of the knife is interrupted over the width of the base, so that at these interruptions of the cutter, the webs joining the successive fleece webs are produced.

The cutting station according to the invention preferably also comprises transport rolls which are arranged upstream of the cutting rolls in the transverse direction of the fleece web. This ensures the uniform feeding of the fleece web into the cutting station.

It should be pointed out that the tearing apart of the sections can take place both directly following the cutting or weakening of the fleece web, that is to say directly following the cutting device according to the invention, but also - which is advantageous - only during the subsequent process sequence, since a fleece web which is still coherent is simpler to process than fleece webs which are already individual and have been separated.

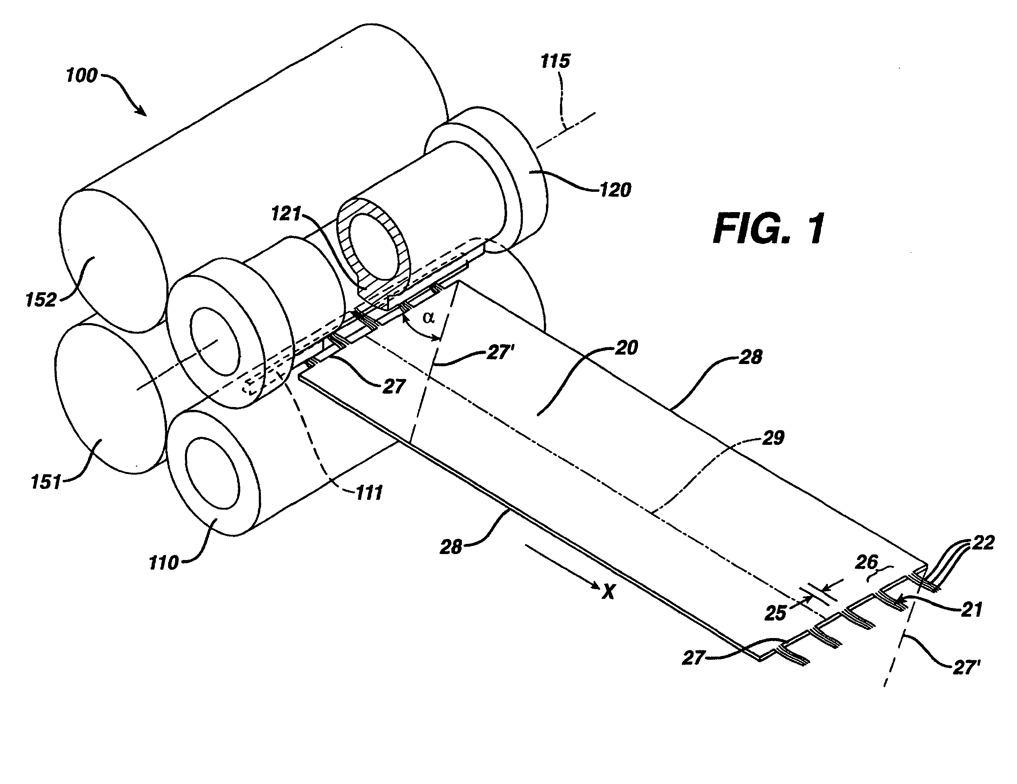

FIG. 1 shown an embodiment of a cutting station 100 according to the invention. The cutting station 100 comprises, in the direction of motion X of a fleece web, a pair of transport rolls 151, 152 which feeds the fleece web uniformly in the direction of motion to cutting rolls 110, 120 arranged behind them.

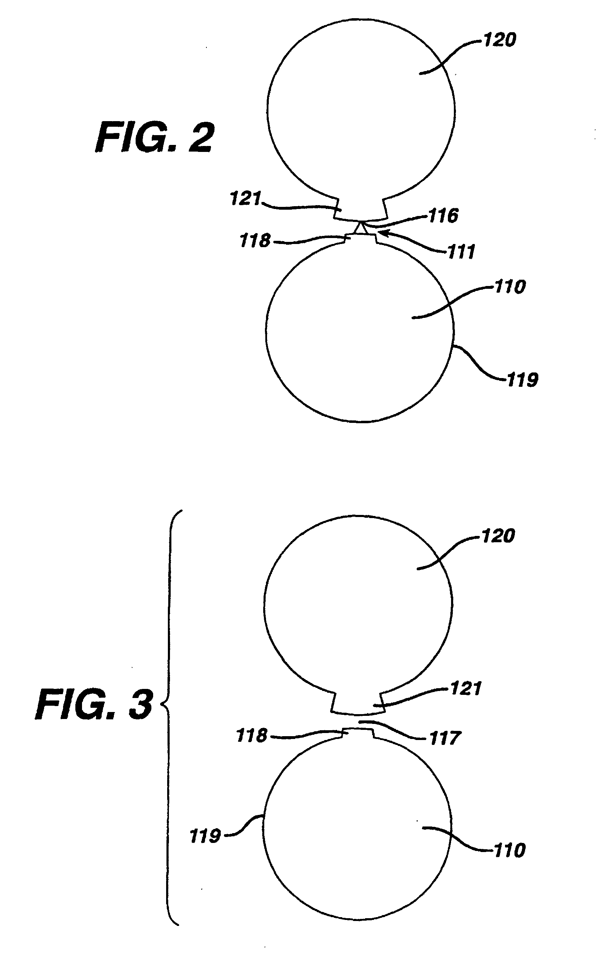

The cutting rolls 110, 120 comprise a knife roll 110 and a backing roll 120. Fitted to the knife roll 110 is a knife 111, which extends essentially over the entire width of the outer surface of the knife roll 110, but at least over the entire width of a fleece web or of a section 20 of the latter. The knife 111 comprises a knife base, which is formed by a thickening as a press jaw 118, and a cutter region with cutters 116 and discontinuities 117, which are arranged alternately. The structure of the knife 111 will be explained in more detail below with reference to FIGS. 2 and 3.

The backing roll 120 has a resilient outer surface, which comes into engagement with the cutters 116 during each complete revolution and cuts through the fleece web in portions 26, while portions 25 are not cut so that extensions 21 are formed.

In Fig. 1 in order to illustrate the result to be achieved with the apparatus according to the invention, a section 20 of the fleece web which has already been separated is illustrated. However as a rule, the fleece web segments 20 still have not been torn apart even after they have passed through the cutting station 100 according to the invention so that there is a still coherent fleece web.

In the regions 25, the fleece web segments 20 has extensions or webs 21 which consist of fibers 22 which have not been cut through by the cutting rolls 110, 120 but are only torn apart subsequently. Between the extensions 21 there are cut regions 26, in which the fleece webs 20 have been separated from one another by the cutting rolls 110, 120. The longitudinal ends 27 essentially form a right angle with a longitudinal axis 29 of the section 20, if the knife 111 is arranged along a generatrix of the cutting roll.

Longitudinal ends 27' of the fleece web 20 are indicated by a dashed line, these ends deviating by an angle α of about 30 from the rectangular arrangement just described with respect to a generatrix of the knife roll 110. (The angle shown in Fig. 1 is illustrated significantly larger to make it clear.) The fleece web 20 then essentially forms a parallelogram.

FIG. 2 shows a cross-section of the embodiment of the cutting rolls 110, 120 shown in FIG. 1 in the region of a cutter 116, the knife 111 of the knife roll 110 and an anvil 121 of the backing roll 120 being in engagement.

The knife ill has a knife base which is thickened and designed as a press jaw 119. This thickening, together with the anvil 121, serves as a pressing device which compresses the fleece web in the vicinity of the cut as it passes through between the cutting rolls 110, 120, so that the and of the fleece web has a lower thickness. Suitable materials are the usual standard materials; in particular, the anvil 121 can be produced fact, slightly resilient material, into which the cutter 116 can penetrate. In a departure from the exemplary embodiment described, the backing roll 120 may also be designed to be completely cylindrical. In this case, one of the two cutting rolls could be arranged to be displaceable to and fro in each case with respect to the other cutting roll in order to carry out the cut.

FIG. 3 shows a cross-section through the cutting rolls 110, 120 analogous to FIG 2, but at the level of a discontinuity 117 in the knife 111. In this region, the fleece web is not cut between the cutting rolls 110, 120 but is only compressed by the narrowed region between the cutting rolls 110, 120, its cross-section is reduced and as a result it is weakened. That region 25 of the fleece web segments 20 in which the extensions 21 are produced and which is shown in FIG. 1 runs through this region.

In the embodiment shown in FIGS. 1 to 3, the knife 111 consists of alternately arranged cutters 116 having a width of 5 mm and recesses with a width of 1 mm. As a result, about 85% of the width of the fleece web is cut by the knife 111, the remaining 15% being subsequently torn. It is to be understood that the knife roll 110 can be designed with a number of knives 111 arranged at identical circumferential angles on its circumference, depending on its diameter. The length of the arc of the circumferential angle in each case corresponds to the length of a fleece web segment 20.

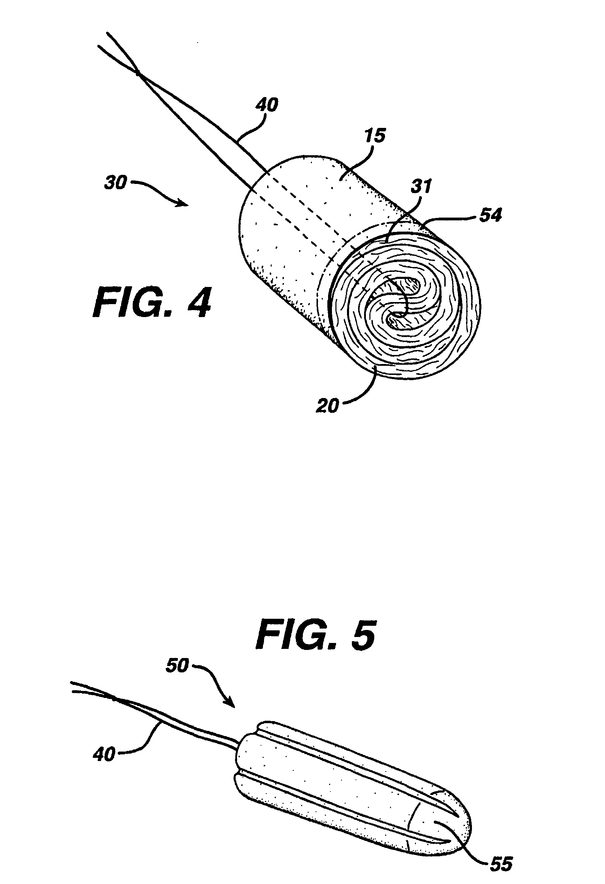

FIG. 4 shows a tampon blank 30 which is produced by winding up the section 20 as shown in FIG. 1. Also shown only schematically is the partly cut and partly torn longitudinal end 31, narrowed in cross section, of the fleece web 20, which is provided with the webs 21 distributed uniformly in the longitudinal direction of the tampon blank 30, these webs being visible in FIG. 1. The reduction in cross section is based on the compression of the section 20 in this region which is brought about by the interaction between the press jaw 118 and the anvil 121 of the backing roll 120. In the other regions, the fleece web 20 has an essentially constant thickness, which corresponds to the thickness of a fleece web supplied.

FIG. 4 shows a withdrawal cord 40, which is provided at a withdrawal end of the tampon blank 30 or of the tampon 50.

A wrapping material 15, which is formed here by a plastic film which is perforated and provided with wax, preferably a polyethylene film, essentially completely encloses the tampon blank 30, but leaves a' portion 54 of the tampon blank 30 free. Following the pressing of the tampon blank 30, this portion 54 forms a dome-shaped insertion end 55 (shown in Fig. 5) of the finished tampon So. This ensures that the insertion and 55 which is not covered by the wrapping material 15 is acted on directly by the body fluid to be absorbed. Thus, as a consequence, the tampon 50 can immediately expand and, as a result, can develop its full absorbency and also undertake the complete protective function for the user.

In the embodiments shown in these figures, a fiber with an irregular, preferably multi-limbed or star-shaped cross-section was used, which significantly improves the capillary action and expansion capacity of the tampon.

The features illustrated in the claims, the description and the drawings may be essential for the invention, both individually and in any combination.

## Claims

1. A tampon (50) having a length comprising:
a) a section (20) of a specific length of a nonwoven fibrous web which has a width approximately corresponding to the length of the tampon (50) and a longitudinal axis (29), the ends of the nonwoven fibrous web section (20) being defined by a reduced cross-section, and the terminal edges (27) of the ends being defined by alternating sections of cut fibers (26) and extended fibers (21) wherein the sections of the cut fibers (26) amount to at least about 70% of the width of the nonwoven fibrous web section (20),
b) a cover strip section (15) thermally bonded to the one end of the nonwoven fibrous web section (20), the cover strip section (15) comprising a liquid-permeable at least partially thermoplastic material, and extending beyond the one end of the nonwoven fibrous web section (20);
c) a withdrawal string (40) extending from the tampon (50);
wherein the nonwoven fibrous web section (20) is wound essentially upon itself to give a substantially round cylindrical tampon blank preform (30) with the cover strip section (15) extending beyond the one end of the nonwoven fibrous web section (20) being thermally bonded to an overlapped portion of the cover strip section (15) to maintain the tampon preform (30) as a cylinder and wherein the cylindrical preform is radially pressed to form a substantially cylindrical compressed tampon (50).

2. The tampon of claim 1 wherein the sections (26) of the cut fibers amount to about 70% to about 90% of the width of the nonwoven fibrous web section (20).

3. The tampon of claim 1 or 2 wherein the sections (26) of the cut fibers amount to about 80% of the width of the nonwoven fibrous web section (20).

4. The tampon of one of the claims 1 to 3, wherein the sections (26) of the cut fibers are aligned along a line that defines an oblique angle α to the longitudinal axis of the non woven fibrous web section.

5. The tampon of claim 4 wherein the oblique angle α is from about 85° to about 89°.

6. The tampon of claim 5 wherein the oblique angle α is about 87°.

7. The tampon of one of the previous claims wherein the cover strip section (15) comprises an apertured film.

8. The tampon of one of the previous claims wherein the cover strip section (15) of the compressed tampon (50) is substantially free of fibers attached thereto.

9. A process for producing a tampon comprising the steps of:
- providing a substantially continuous fleece web comprising fibers;
- cutting the continuous fibrous web essentially over its width to form a fleece web segment (20) connected to the continuous fibrous web through a plurality of uncut extensions (25), wherein the sections (26) of the cut fibers amount to at least about 70% of the width of the fibrous web;
- tearing apart the individual portions of the web to form separate fleece web section (20);
- winding up the separate fleece web section (20) to form a tampon blank (30); and
- compressing the tampon blank (30) into the final shape of the tampon (50).

10. The process as claimed in claim 9, wherein at least a portion of the fleece web segment (20) at the cut end is weakened.

11. The process as claimed in claim 10, wherein, the weakened portion of the fleece web segment (20) has a reduced cross-sectional area.

12. The process as claimed in one of the claims 9 to 11, wherein the cutting defines a line at a right angle to the longitudinal axis of the continuous fibrous web.

13. The process as claimed in claim 9 to 11, wherein the cutting defines a line which is oblique to the longitudinal axis of the continuous fibrous web.

14. The process as claimed in claim 13, wherein the oblique angle α is from about 85° to about 89°.

15. The process as claimed in one of the claims 9 to 14, which further comprises the step of attaching a wrapping material to the individual fleece segment wherein the wrapping material forms an outer cylindrical surface of the tampon blank (30) after the winding of the individual fleece segment.

16. The process as claimed in one of the claims 9 to 15, wherein the continuous fibrous web comprises a mixture of natural and artificial fibers.

17. The process as claimed in one of the claims 9 to 16, wherein the continuous fibrous web comprises fibers having a multi-limbed cross-section.

18. A process as claimed in one of the claims 9 to 17, wherein the step of tearing apart the individual portions of the web to form a separate fleece web section comprises pulling away a fleece web segment (20) from the continuous fibrous web.

19. An apparatus for producing a tampon, the apparatus comprising:
- means for providing a substantially continuous fleece web comprising fibers,
- a cutting station (100) having a knife roll (110) with at least one knife (111), and a backing roll (120) wherein the at least one knife (111) being arranged on at least one portion of an outer surface of the knife roll and the knife further having, at least in part, discontinuities (117) for the webs of the fleece webs, the knife extending essentially over an entire width of the fleece web to be cut, wherein the discontinuities (117) of the knife amount to a maximum of about 30% of the cutting length of the knife;
- means for tearing apart the individual portions of the web to form a separate fleece web section (20);
- means for winding up the separate fleece web section to form a tampon blank (30); and
- means for compressing the tampon blank (30) radially into the final shape of the tampon (50).

20. The apparatus as claimed in claim (19), wherein the at least one knife (111) is arranged on the outer surface of the knife roll (110) essentially along a generatrix of the knife roll (110).

21. The apparatus as claimed in claim 19, wherein the knife (111) forms an angle α with the generatrix of the knife roll (110).

22. The apparatus as claimed in claim 21, wherein the angle α lies in the range from 1° to 5°

23. The apparatus as claimed in one of the claims 19 to 22, wherein the cutting station (100) further comprises a weakening device.

24. The apparatus as claimed in claim 23, wherein the knife (111) comprises a knife base and the weakening device comprises
- a thickened portion of the knife base, forming a press jaw in the vicinity of the cover of the knife roll (110); and
- a region of the backing roll (120).

25. The apparatus as claimed in claim 24, which further comprises a cutter arranged and configured on the press jaw.

26. The apparatus as claimed in claim 25 wherein the cutter of the knife is interrupted over the width of the base.

## Patentansprüche

1. Tampon (50) mit einer Länge umfassend:
a) einen Abschnitt (20) bestimmter Länge einer Faservliesbahn, die eine der Länge des Tampons (50) annähernd entsprechende Breite und eine Längsachse (29) aufweist, wobei die Enden des Faservliesbahnabschnitts (20) durch einen verminderten Querschnitt definiert sind und die Abschlußkanten (27) der Enden durch abwechselnde Bereiche von geschnittenen Fasern (26) und weiterführenden Fasern (21) definiert sind, wobei die Abschnitte von geschnittenen Fasern zumindest ca. 70% der Breite des Faservliesbahnabschnitts (20) ausmachen,
b) einen Deckstreifenabschnitt (15), der durch Wärmebehandlung mit dem einen Ende des Faservliesbahnabschnitts (20) verbunden ist, wobei der Deckstreifenabschnitt (15) ein flüssigkeitsdurchlässiges, zumindest teilweise thermoplastisches Material umfaßt und sich über das eine Ende des Faservliesbahnabschnitts (20) erstreckt;
c) ein Rückholband (40), das sich von dem Tampon (50) erstreckt;
wobei der Faservliesbahnabschnitt (20) im wesentlichen um sich selbst gewickelt ist, um einen im wesentlichen runden, zylindrischen Tamponvorformling (30) zu bilden, wobei sich der Deckstreifenabschnitt (15) über das eine Ende des Faservliesbahnabschnitts (20) erstreckt, der durch Wärmebehandlung mit einem Überlappbereich des Deckstreifenabschnitts (15) verbunden ist, um den Tamponvorformling (30) als Zylinder zu erhalten, wobei der zylindrische Vorformling radial zusammengedrückt ist, um ein im wesentlichen zylindrisches, zusammengedrücktes Tampon (50) zu formen.

2. Tampon nach Anspruch 1, bei dem die Abschnitte (26) von geschnittenen Fasern ca. 70% bis ca. 90% der Breite des Faservliesbahnabschnitts (20) ausmachen.

3. Tampon nach Anspruch 1 oder 2, bei dem die Abschnitte (26) von geschnittenen Fasern ca. 80% der Breite des Faservliesbahnabschnitts (20) ausmachen.

4. Tampon nach einem der Ansprüche 1 bis 3, bei dem die Abschnitte (26) von geschnittenen Fasern linear ausgerichtet sind, wobei ein spitzer Winkel α zur Längsachse des Faservliesbahnabschnitts definiert ist.

5. Tampon nach Anspruch 4, bei dem der spitze Winkel α ca. 85° bis ca. 89° ist.

6. Tampon nach Anspruch 5, bei dem der spitze Winkel α ca. 87° ist.

7. Tampon nach einem der vorangegangenen Ansprüche, bei dem der Deckstreifenabschnitt (15) eine mit Löcher versehene Folie umfaßt.

8. Tampon nach einem der vorangegangenen Ansprüche, bei dem der Deckstreifenabschnitt (15) des zusammengedrückten Tampons im wesentlichen frei von daran anhängenden Fasern ist.

9. Verfahren zum Herstellen eines Tampons, wobei:
- eine im wesentlichen kontinuierliche Vliesbahn mit Fasern bereitgestellt wird;
- die kontinuierliche Faserbahn im wesentlichen über deren Breite geschnitten wird, um ein Vliesbahnsegment (20) zu bilden, das mit der kontinuierlichen Faserbahn über mehrere ungeschnittene Stege (25) verbunden ist, wobei die Abschnitte (26) von geschnittenen Fasern zumindest ca. 70% der Breite der Faserbahn ausmachen;
- die einzelnen Abschnitte der Bahn auseinander gerissen werden, um einen separaten Vliesbahnabschnitt (20) zu bilden;
- der separate Vliesbahnabschnitt (20) gewickelt wird, um einen Tamponrohling (30) zu bilden; und
- der Tamponrohling (30) in die Endform des Tampons (50) zusammengedrückt wird.

10. Verfahren nach Anspruch 9, bei dem zumindest ein Abschnitt des Vliesbahnsegments (20) am geschnittenen Ende geschwächt wird.

11. Verfahren nach Anspruch 10, bei dem der geschwächte Abschnitt des Vliesbahnsegments (20) einen verminderten Querschnittsbereich aufweist.

12. Verfahren nach einem der Ansprüche 9 bis 11, bei dem der Schnitt eine Linie in einem rechten Winkel zur Längsachse der kontinuierlichen Faserbahn definiert.

13. Verfahren nach einem der Ansprüche 9 bis 11, bei dem der Schnitt eine Linie definiert, die schräg zur Längsachse der kontinuierlichen Faserbahn liegt.

14. Verfahren nach Anspruch 13, bei dem der spitze Winkel α ca. 85° bis ca. 89° ist.

15. Verfahren nach einem der Ansprüche 9 bis 14, bei dem ein Einhüllmaterial an das einzelne Vliessegment angebracht wird, wobei das Einhüllmaterial eine äußere zylindrische Fläche des Tamponrohlings (30) nach dem Wickeln des einzelnen Vliessegments bildet.

16. Verfahren nach einem der Ansprüche 9 bis 15, bei dem die kontinuierliche Faserbahn eine Mischung aus Natur- und Kunstfasern umfaßt.

17. Verfahren nach einem der Ansprüche 9 bis 16, bei dem die kontinuierliche Faserbahn Fasern umfaßt, die einen vielgliedrigen Querschnitt aufweisen.

18. Verfahren nach einem der Ansprüche 9 bis 17, bei dem beim Auseinanderreißen der einzelnen Abschnitte der Bahn zum Bilden eines separaten Vliesbahnabschnitts ein Vliesbahnsegment (20) von der kontinuierlichen Faserbahn weggezogen wird.

19. Vorrichtung zum Herstellen eines Tampons, umfassend:
- eine Einrichtung zum Bereitstellen einer im wesentlichen kontinuierlichen Vliesbahn mit Fasern,
- eine Schneidestation (100) mit einer Messerwalze (110), die zumindest ein Messer (111) und eine Stützwalze (120) aufweist, wobei das zumindest eine Messer (110) an zumindest einem Abschnitt einer Außenfläche der Messerwalze angeordnet ist und das Messer außerdem zumindest teilweise Unterbrechungen (117) für die Vliesbahnen aufweist, wobei sich das Messer im wesentlichen über die gesamte Breite der zu schneidenden Vliesbahn erstreckt, wobei die Unterbrechungen (117) des Messers maximal ca. 30% der Schneidenlänge des Messers ausmachen;
- eine Einrichtung zum Auseinanderreißen der einzelnen Abschnitte der Bahn zum Bilden eines separaten Vliesbahnabschnitts (20);
- eine Einrichtung zum Wickeln des separaten Vliesbahnabschnitts zum Bilden eines Tamponrohlings (30); und
- eine Einrichtung zum radialen Zusammendrücken des Tamponrohlings (30) in die Endform des Tampons (50).

20. Vorrichtung nach Anspruch 19, bei der das zumindest eine Messer (111) an der Außenfläche der Messerwalze (110) im wesentlichen längs einer Kante der Messerwalze (110) angeordnet ist.

21. Vorrichtung nach Anspruch 19, bei der das Messer (111) einen Winkel α bezüglich der Kante der Messerwalze (110) bildet.

22. Vorrichtung nach Anspruch 21, bei dem der Winkel α in einem Bereich von 1° bis 5° liegt.

23. Vorrichtung nach einem der Ansprüche 19 bis 22, bei dem die Schneidestation (100) außerdem eine Schwächungseinrichtung umfaßt.

24. Vorrichtung nach Anspruch 23, bei der das Messer (111) eine Messerbasis umfaßt und die Schwächungseinrichtung
- einen verstärkten Abschnitt der Messerbasis, der eine Druckbacke in der Nähe der Abdeckung der Messerwalze (110) bildet; und
- einen Bereich der Stützwalze (120) umfaßt.

25. Vorrichtung nach Anspruch 24, die außerdem eine Schneideinrichtung umfaßt, die an der Druckbacke angeordnet und konfiguriert ist.

26. Vorrichtung nach Anspruch 25, bei dem die Schneideinrichtung des Messers über die Breite der Basis unterbrochen ist.

## Revendications

1. Tampon (50) doté d'une longueur comprenant :
a) une section (20) d'une longueur spécifique d'une bande fibreuse non tissée qui a une largeur qui correspond approximativement à la longueur du tampon (50) et un axe longitudinal (29), les extrémités de la section de bande fibreuse non tissée (20) étant définies par une section transversale réduite, et les bords terminaux (27) des extrémités étant définis par des sections alternées de fibres coupées (26) et de fibres étendues (21), dans lequel les sections de fibres coupées (26) s'élèvent au moins à environ 70% de la largeur de la section de bande fibreuse non tissée (20),
b) une section de bande de recouvrement (15) thermiquement collée à la une extrémité de la section de bande fibreuse non tissée (20), la section de bande de recouvrement (15) comprenant un matériau perméable au liquide au moins partiellement thermoplastique, et s'étendant au-delà de la une extrémité de la section de bande fibreuse non tissée (20) ;
c) une ficelle de retrait (40) qui s'étend depuis le tampon (50) ;
dans lequel la section de bande fibreuse non tissée (20) est essentiellement enroulée sur elle-même pour donner une préforme d'ébauche de tampon cylindrique sensiblement arrondie (30) avec la section de bande de recouvrement (15) qui s'étend au-delà de la une extrémité de la section de bande fibreuse non tissée (20) qui est thermiquement collée à une partie de chevauchement de la section de bande de recouvrement (15) pour maintenir la préforme du tampon (30) comme un cylindre et dans lequel la préforme cylindrique est pressée de manière radiale pour former un tampon compressé sensiblement cylindrique (50).

2. Tampon selon la revendication 1 dans lequel les sections (26) des fibres coupées s'élèvent à environ 70% à 90% de la largeur de la section de bande fibreuse non tissée (20).

3. Tampon selon la revendication 1 ou 2 dans lequel les sections (26) des fibres coupées s'élèvent à environ 80% de la largeur de la section de bande fibreuse non tissée (20).

4. Tampon selon l'une quelconque des revendications 1 à 3, dans lequel les sections (26) des fibres coupées sont alignées le long d'une ligne qui définit un angle oblique α par rapport à l'axe longitudinal de la section de bande fibreuse non tissée.

5. Tampon selon la revendication 4, dans lequel l'angle oblique α est compris entre environ 85° et environ 89°.

6. Tampon selon la revendication 5, dans lequel l'angle oblique α est d'environ 87°.

7. Tampon selon l'une quelconque des revendications précédentes dans lequel la section de bande de recouvrement (15) comprend un film ouvert.

8. Tampon selon l'une quelconque des revendications précédentes dans lequel la section de bande de recouvrement (15) du tampon comprimé (50) est sensiblement dépourvue de fibres fixées à celle-ci.

9. Procédé pour produire un tampon comprend les étapes consistant à :
proposer une bande de molleton sensiblement continue qui comprend des fibres ;
couper la bande fibreuse continue essentiellement sur sa largeur pour former un segment de bande de molleton (20) raccordé à la bande fibreuse continue à travers une pluralité d'extensions non coupées (25), dans laquelle les sections (26) des fibres coupées s'élèvent au moins à environ 70% de la largeur de la bande fibreuse ;
déchirer des parties individuelles de la bande pour former la section de bande de molleton (20) ;
enrouler la section de bande de molleton séparée (20) pour former une ébauche de tampon (30) ; et
comprimer l'ébauche de tampon (30) dans la forme définitive du tampon (50).

10. Procédé selon la revendication 9, dans lequel au moins une partie du segment de bande de molleton (20) est affaiblie au niveau de l'extrémité coupée.

11. Procédé selon la revendication 10, dans lequel la partie affaiblie du segment de bande de molleton (20) possède une zone de section transversale réduite.

12. Procédé selon l'une quelconque des revendications 9 à 11, dans lequel la coupe définit une ligne en angle droit par rapport à l'axe longitudinal de la bande fibreuse continue.

13. Procédé selon les revendications 9 à 11, dans lequel la coupe définit une ligne qui est oblique par rapport à l'axe longitudinal de la bande fibreuse continue.

14. Procédé selon la revendication 13, dans lequel l'angle oblique α est compris entre environ 85° et environ 89°.

15. Procédé selon l'une quelconque des revendications 9 à 14, qui comprend en outre l'étape consistant à fixer un matériau enveloppant sur le segment de molleton individuel dans lequel le matériau enveloppant forme une surface cylindrique externe de l'ébauche de tampon (30) après l'enroulement du segment de molleton individuel.

16. Procédé selon l'une quelconque des revendications 9 à 15, dans lequel la bande fibreuse continue comprend un mélange de fibres naturelles et artificielles.

17. Procédé selon l'une quelconque des revendications 9 à 16, dans lequel la bande fibreuse continue comprend des fibres présentant une section transversale à plusieurs membres.

18. Procédé selon l'une quelconque des revendications 9 à 17, dans lequel l'étape consistant à déchirer les parties individuelles de la bande pour former une section de bande de molleton séparée comprend l'étape consistant à tirer un segment de bande de molleton (20) de la bande fibreuse continue.

19. Appareil pour produire un tampon, l'appareil comprenant :
des moyens pour proposer une bande de molleton sensiblement continue comprenant des fibres,
une station de coupe (100) doté d'un cylindre à lames (110) avec au moins une lame (111), et un contre-rouleau (120), dans lequel la au moins une lame (111) est agencée sur au moins une partie d'une surface externe du cylindre à lames et la lame présentant en outre, au moins en partie des discontinuités (117) pour les bandes de bandes de molleton, la lame s'étendant sensiblement sur une largeur totale de la bande de molleton à couper, dans lequel les discontinuités (117) de la lame s'élèvent à un maximum d'environ 30% de la longueur de coupe de la lame ;
des moyens pour déchirer les parties individuelles de la bande pour former une section de bande de molleton séparée (20) ;
des moyens pour enrouler la section de bande de molleton séparée pour former une ébauche de tampon (30) ; et
des moyens pour comprimer l'ébauche de tampon (30) de manière radiale dans la forme définitive du tampon (50).

20. Appareil selon la revendication 19, dans lequel au moins une lame (111) est agencée sur la surface externe du cylindre à lames (110) essentiellement le long d'une génératrice du cylindre à lames (110).

21. Appareil selon la revendication 19, dans lequel la lame (111) forme un angle α avec la génératrice du cylindre à lames (110).

22. Appareil selon la revendication 21, dans lequel l'angle α est compris entre 1° et 5°.

23. Appareil selon l'une quelconque des revendications 19 à 22, dans lequel la station de coupe (100) comprend en outre un dispositif d'affaiblissement.

24. Appareil selon la revendication 23, dans lequel la lame (111) comprend une base de lame et le dispositif d'affaiblissement comprend :
une partie épaissie de la base de lame, formant une mâchoire de presse à proximité de la couverture du cylindre à lames (110) ; et
une région de contre-rouleau (120).

25. Appareil selon la revendication 24 qui comprend en outre un dispositif de coupe agencé et configuré sur la mâchoire de presse.

26. Appareil selon la revendication 25 dans lequel le dispositif de coupe de la lame est interrompu sur la largeur de la base.
